# EUROPÄISCHE PATENTSCHRIFT

(11) **EP 0 504 348 B1**
(45) Veröffentlichungstag und Bekanntmachung des Hinweises auf die Patenterteilung: **12.07.1995**
(21) Anmeldenummer: 91917092.8
(22) Anmeldetag: 01.10.1991
(51) Int. Cl.: A61N 1/18, A61N 1/20

(54) **PFLASTERANORDNUNG ZUR GALVANISCHEN BEHANDLUNG**
PLASTER ARRANGEMENT FOR GALVANIC TREATMENT
DISPOSITIF A PATE CONDUCTRICE POUR ELECTROTHERAPIE

(30) Priorität: 10.10.1990 DE 4032109; 06.05.1991 DE 4114677
(43) Veröffentlichungstag der Anmeldung: 23.09.1992
(73) Patentinhaber: BERGNER, Mario, D-89081 Ulm-Söflingen (DE)
(72) Erfinder: BERGNER, Mario, D-89081 Ulm-Söflingen (DE)
(74) Vertreter: Fritz, Edmund Lothar, Dipl.-Chem.
(86) Internationale Anmeldenummer: DE9100772
(87) Internationale Veröffentlichungsnummer: WO9206736

(56) Entgegenhaltungen:
- WO-A-90/09205
- CH-A- 653 897
- DE-A- 3 309 841

## Beschreibung

Die Erfindung betrifft eine Pflasteranordnung elektrisch verbundener Elektroden aus verschiedenen Metallen am menschlichen Körper zur elektrischen Beeinflussung elektrisch tätiger Strukturen des Organismus.

Bei Zuständen von Muskelverspannungen und Nervenwurzelreizungen besonders im Bereich der Wirbelsäule wird der Elektrostrombehandlung zunehmend der Vorzug gegeben. Die Abneigung gegenüber einer entsprechenden medikamentösen Therapie steigt.
Der Anwendung eines Gleichstromes durch das zu behandelnde Gewebe ( stabile Galvanisation ) kommt eine große Bedeutung zu. In der Praxis werden hierzu größere stationäre Geräte verwendet, die auch für andere Stromformen ausgelegt sind ( sogenannte Reizstromgeräte ). Zur ambulanten bzw. mittelfristigen Daueranwendung befinden sich kleine tragbare und batteriebetriebene Geräte ( sogenannte TENS-Geräte ) auf dem Markt. Während eine längerfristige Anwendung mit den stationären Geräten unmöglich ist, ist sie mit den tragbaren Geräten wegen der doch nötigen Größe immer noch unbequem.

Die Behandlung von Muskelverspannungen aller Art mit einem vergleichsweise kleinem Dauerstrom im »A - Bereich hat sich als sehr wirkungsvoll erwiesen. Aus diesem Grunde sind verschiedene Anordnungen beschrieben worden, bei denen zur Erzeugung eines Stromflusses durch das zu behandelnde Körpergewebe das Spannungsgefälle zwischen zwei unterschiedlich edlen Metallen im Kontakt mit dem Hauttranspirationselektrolyten genutzt wurde. Die Anordnung nach PS GB 3276(1904) benötigt zum Erreichen eines ausreichenden Stromflusses allerdings sehr große Hautelektroden. Nach der CH-PS 171866 wird die galvanische Spannung mittels verschiedener Metallelektroden sowie des Hauttranspirationselektrolyten und/oder verschiedener mit Elektrolyt getränkter Stofflagen erzeugt. Auch in diesem Zusammenhang vorgeschlagene Klebstoffbeschichtungen führen zu Lösungen, die praktisch inklusive teurer Metallelektroden kurzfristig nutzbare Einmalartikel sind.

Die GB-PS Nr. 288 von 1904 beschreibt übereinander geschichtete Scheiben aus abwechselnd edlerem und unedlerem Metall, wobei zwar von galvanischen Zwischenlagen zwischen Zink und Kupfer gesprochen wird, die Form eines geschlossenen Stromkreises aber nicht geklärt wird.
Die bisher vorgeschlagenen technischen Lösungen konnten sich wegen mangelnder Wirksamkeit oder unpraktischer Anwendung nicht durchsetzen.

Die Erfindung bezweckt eine Erhöhung der Stromdichte bei möglichst klein gehaltenen Hautkontaktelektroden, eine unkomplizierte Handhabbarkeit sowie kostengünstige Herstellung und damit auch Anwendung.
Dabei sollte ein auf dem Markt bereits vorhandenes Produkt als billiger Einmalartikel neben der Fixierung der Elektrodenanordnung auf der Körperoberfläche zusätzlich der Schaffung weiterer galvanischer Elemente zwecks Erhöhung der Spannung dienen. Der erfindungsgemäße Gegenstand, der ausschließlich aus den Metallelektroden, als dem eigentlich teuren Anteil des galvanischen Systems und einer Randisolierung besteht, sollte wiederverwendbar und von relativ langer Lebensdauer sein.

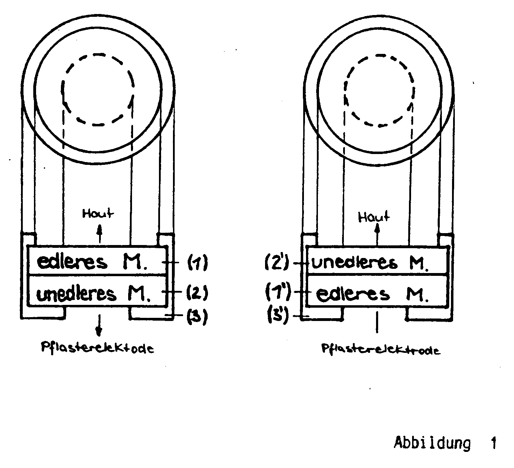

Erfindungsgemäß wird diese Aufgabe dadurch gelöst, daß zunächst beidseits der kranken Körperregion je eine vorzugsweise runde Flächenelektrode aus unterschiedlich edlen Metallen in bekannter Weise angeordnet wird. Auf diese beiden Hautkontaktelektroden ( 1,2' ) ist umseitig bzw. körperfern jeweils eine weitere Metallelektrode ( 2,1' ) aufgetragen, wobei die Metalle dieser Elektroden denen der beiden Hautelektroden ( 1,2' ) entsprechen können, nach der Polarität im Sinne der elektrochemischen Spannungsreihe aber stets entgegengesetzt anzuordnen sind. Die beiden so entstandenen kompakten Doppelmetallelektroden ( 1,2 und 2',1' ) müssen gegen einen Elektrolytkurzschluß am Rand isoliert sein ( 3,3' ) ( Abbildung 1 ).
So entstehen zwei Doppelmetallscheiben mit Randisolation, die leicht herstellbar sind, die den teuren Anteil ( Metall ) eines elektrogalvanischen Systems in kompaktester Form darstellen, die wiederverwendbar sowie von langer Lebensdauer sind und die so gestaltet werden, daß sie unter Verwendung von handelsüblichen Elektrokardiographieelektrodenpflastern ( 5,5' ) über ein Klipkontaktkabel ( 8 ) zu einem geschlossenen biogalvanischen System ergänzt werden können.

Dieses den erfindungsgemäßen Gegenstand nicht betreffende Elektrodenpflaster ( 4,5,6,7 bzw. 4',5',6',7' ) enthält somit alle Elemente des biogalvanischen Systems, die nur zum Einmalgebrauch geeignet oder von begrenzter Wirksamkeit sind ( Pflaster 5,5'; Elektrodengel 6,6' ). Eine sehr wirkungsvolle und zudem wasserdichte Fixierung ist möglich.
Darüberhinaus entsteht so jeweils zwischen einer körperfernen Metallelektrode ( 2,1' ) und der Silberelektrode eines Pflasters ( 4,4' ) mit dem zugehörigen Silberchloridgelkissen ( 6,6' ) ein zusätzliches galvanisches Element in Reihe zu dem in der Literatur bereits genannten galvanischen Element aus zwei verschiedenen Metallen ( 1,2' ) auf der Haut. Auf diesem Wege werden bei geeigneter Auswahl der Metalle drei galvanische Elemente gebildet, deren Reihenschaltung zu einem bedeutendem Spannungszuwachs gegenüber bisherigen Anordnungen führt.
Zur Schließung des elektrischen Stromkreises zwischen den beiden Druckknopfkontakten ( 7,7' ) der Elektrodenpflaster wird vorzugsweise ein dehnungselastisches, elektrisch leitendes Kabel ( 8 ) mit an den Enden angebrachten Federkontaktösen ( 9,9' ) verwendet. Mit einem in dieses Kabel eingearbeiteten regelbaren Widerstand wäre eine Stromregulierung durch den Anwender möglich.

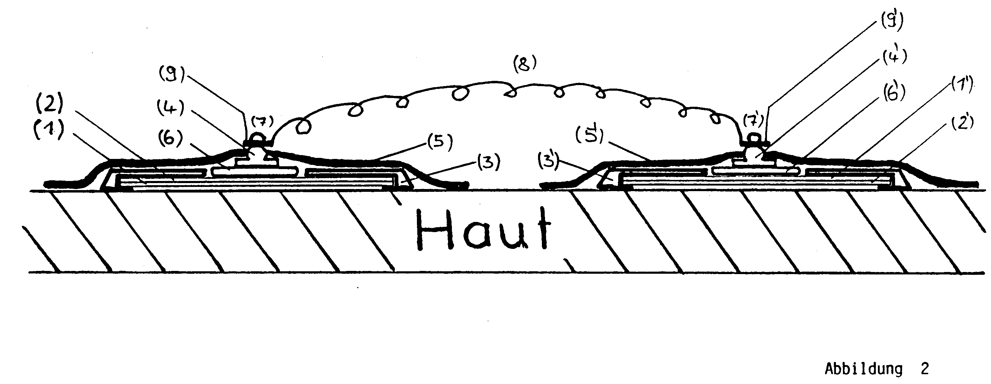

Abbildung 2 verdeutlicht den Aufbau des oben beschriebenen Stromkreises wie folgt: Haut - ( 1 ) - ( 2 ) - ( 6 ) - ( 4 ) - ( 7 ) - ( 9 ) - ( 8 ) - ( 9') - ( 7' ) - ( 4' ) - ( 6' ) - ( 1' ) - ( 2' ) - Haut .

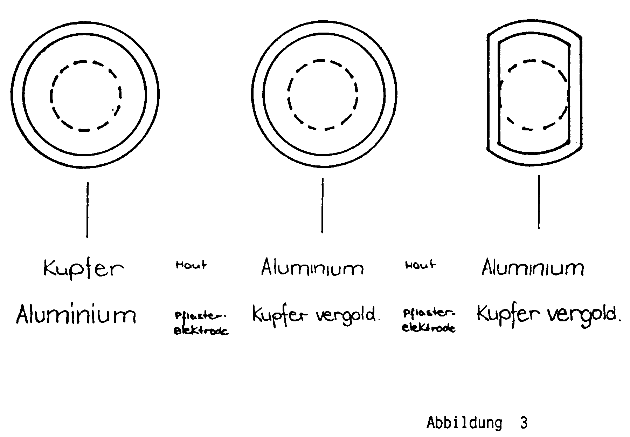

Durch Abbildung 3 soll ein Ausführungsbeispiel gegeben werden.
Mit dem Ziel, muskuläre Verspannungen insbesondere in der geraden Rückenmuskulatur und die damit bei vielen Menschen häufig verbundenen Rückenschmerzen und Nackenverspannungen zu beseitigen, kann die oben beschriebene Anordnung aus Elektrokardiographieelektrodenpflaster und Doppelelektrode vorzugsweise beidseits der schmerzenden Region in Längsrichtung über der Wirbelsäule angebracht und für längere Zeit ( mehrere Tage sind möglich ) belassen werden.
Bei Verwendung geeigneter Metalle aus der gesamten Breite der elektrochemischen Spannungsreihe können sehr wirkungsvolle Ströme erzeugt werden. Empfehlenswert wäre folgende Reihe von Metallen im beschriebenen galvanischen Dreifachsystem ( Abbildung 2 und 3 ) :
Aluminium (1) - Kupfer vergoldet (2) - Silberchloridgel (6) - Silber (4) - Silber (4') - Silberchl.gel (6') - Alu. (1') - Kupfer verg. (2')-Haut.

Handelstechnisch wäre an ein Set mit drei Doppelelektroden zu denken. Zwei runde für die Applikation über der Brustwirbelsäule und Lendenwirbelsäule und einer schmaleren zur Befestigung direkt am Nackenhaaransatz bei Beschwerden im Schulter - Nacken- Bereich.
Bei der letzteren Anwendungsmöglichkeit findet entsprechend zusätzlich eine der runden Doppelelektroden Verwendung ( Abbildung 3 ).
Das dehnungselastische und elektrisch leitende Kabel kann aus einem Gummizug mit aufgetragenem Kupferdrahtwickel sowie den an den Enden angebrachten Klipkontaktösen ( 9,9' ) gefertigt werden.
Für die Anwendung am Patienten mit empfindlicher Haut besteht die Möglichkeit, die beiden Hautkontaktelektroden ( 1,2' ) durch solche aus gleichem, hautverträglichem und elektrisch leitendem Material zu ersetzen. Da das galvanische Element aus diesen beiden Elektroden gegenüber den beiden anderen galvanischen Elementen ( 2,6,4 und 1',6',4' ) den kleineren Spannungsanteil liefert, wird an dem Grundkonzept der Erfindung nichts verändert.

## Patentansprüche

1. Pflasteranordnung zur galvanischen Behandlung, bei der eine Behandlungselektrode mindestens zwei, ein galvanisches Element bildende, verschieden edle Stoffe umfaßt,
dadurch gekennzeichnet,
a) daß die Silberelektrode (4) mit dem zugehörigen Silberchloridgelkissen (6) eines ersten Elektrokardiographieelektrodenpflasters (4 7) mit einer Metallfläche zweier, als Scheibe ausgebildeter, formschlüssig und elektrisch leitend miteinander verbundener, verschieden edler Metalle (2,1) in Kontakt gebracht ist und eine erste Pflasterelektrode bildet,
b) daß die Silberelektrode (4') mit dem zugehörigen Silberchloridgelkissen (6') eines zweiten Elektrokardiographieelektrodenpflasters (4'-7') mit einer Metallfläche zweier weiterer, als Scheibe ausgebildeter, formschlüssig und elektrisch leitend miteinander verbundener, verschieden edler Metalle (1',2') in Kontakt gebracht ist und eine zweite Pflasterelektrode bildet, wobei die Reihenfolge der Anordnung der beiden verschieden edlen Metalle (1',2') hinsichtlich ihrer Edelkeit in bezug auf die Silberelektrode (4,4') des zugehörigen Elektrokardiographieelektrodenpflasters vertauscht ist,
c) und daß zur Schließung des Behandlungsstromkreises zwischen den beiden Druckknopfkontakten (7,7' ) der Elektrokardiographieelektrodenpflaster ein Kabel (8) mit Federkontaktoesen (9,9') angebracht ist.

2. Pflasteranordnung nach Anspruch 1,
dadurch gekennzeichnet,
daß am Rand der als Scheibe ausgebildeten Metalle (2,1 und 1',2') eine Isolierung (3,3') angebracht ist.

3. Pflasteranordnung nach Anspruch 1 oder 2,
dadurch gekennzeichnet, daß in das Kabel (8) ein veränderbarer elektrischer Widerstand eingearbeitet ist.

4. Pflasteranordnung nach einem der vorangehenden Ansprüche, dadurch gekennzeichnet, daß das Kabel (8) dehnungselastisch ist.

5. Pflasteranordnung nach einem der vorhergehenden Ansprüche, dadurch gekennzeichnet, daß die jeweils der Haut zugewandten Scheiben aus Metall (1, 2') der ersten Pflasterelektrode und der zweiten Pflasterelektrode jeweils aus dem gleichen hochwertigen gut hautverträglichen und elektrisch leitenden Material bestehen.

6. Pflasteranordnung nach einem der vorhergehenden Ansprüche, dadurch gekennzeichnet, daß diese einen mit einem Elektrolyten getränkten Träger, vorzugsweise ein angefeuchtetes, mit Soda getränktes Fließpapier, umfaßt, das zwischen den jeweils der Haut zugewandten Scheiben aus Metall (1, 2') und der Haut angeordnet ist.

7. Pflasteranordnung nach einem der vorhergehenden Ansprüche, dadurch gekennzeichnet, daß die Pflasteranordnung aus mehr als zwei Pflasterelektroden besteht.

## Claims

1. Plaster arrangement for galvanic treatment in which a treatment electrode comprises at least two different precious materials forming a galvanic element,
characterised in
a) that the silver electrode (4) with the associated silver chloride gel pad (6) of a first electrocardiographie electrode plaster (4, 7) is brought into contact with a metal surface of two differently precious metals (2, 1) being shaped as a disc and being connected to each other in a form-locking and electrically conducting manner so as to form a first plaster electrode,
b) that the silver electrode (4') with the associated silver chloride gel pad (6') of a second electrocardiographie electrode plaster (4', 7') is brought into contact with a metal surface of two further differently precious metals (1', 2') being shaped as a disc and being connected to each other in a form-locking and electrically conducting manner so as to form a second plaster electrode, the order of the arrangement of the two differently precious metals (1', 2') being interchanged as regards their preciousness relative to the silver electrode (4, 4') of the associated electrocardiographie electrode plaster,
c) and that for closing the treatment circuit between the two push button contacts (7, 7') of the electrocardiographie electrode plasters a cable (8) having spring contact lugs (9, 9') is arranged.

2. Plaster arrangement as claimed in claim 1, characterised in that at the edge of the metals (2, 1 and 1', 2') shaped as a disc there is arranged an insulation (3, 3').

3. Plaster arrangement as claimed in claim 1 or 2, characterised in that a variable electric resistor is integrated in the cable (8).

4. Plaster arrangement as claimed in any one of the preceding claims, characterised in that the cable (8) is elastically ductile.

5. Plaster arrangement as claimed in any one of the preceding claims, characterised in that the discs of metal (1, 2') of the first plaster electrode and the second plaster electrode facing the skin are in each case made of the same highquality material which is skin consistent and electrically conductive.

6. Plaster arrangement as claimed in any one of the preceding claims, characterised in that it comprises a carrier soaked with an electrolyte, preferably a moistened blotting paper soaked with soda, which is arranged between the discs of metal (1, 2') facing the skin and the skin itself.

7. Plaster arrangement as claimed in any one of the preceding claims, characterised in that the plaster arrangement consists of more than two plaster electrodes.

## Revendications

1. Dispositif d' emplâtre pour le traitement galvanique dans lequel un électrode de traitement comprend au moins deux matières précieuses en nature différent qui forment un élément galvanique, caractérisé en ce
a) que l'électrode d'argent (4) avec le coussin en gel deutochloride d'argent (6) associé d'un premier emplâtre à électrode d'électrocardiographie (4, 7) est met en contact avec une surface métallique de deux métaux précieux (2, 1) en nature différent étant en forme d'un disque, conjoints en manière juste et conductible à l'électricité, et forme une première électrode en forme d'emplâtre,
b) que l'électrode d'argent (4') avec le coussinet en gel deutochloride d'argent (6') associé d'un deuxième emplâtre à électrode d'électrocardiographie (4', 7') est met en contact avec une surface métallique de deux autres métaux précieux (1', 2') en nature différent étant en forme d'un disque, conjoints en manière juste et conductible à l'électricité et forme une deuxième électrode en forme d' emplâtre, l'ordre de la position des deux métaux précieux (1', 2') en nature différent étant échanger à l'égard de leur noblesse relativement à l'électrode d'argent (4, 4') d'emplâtre à électrode associé d'électrocardiographie,
c) et que pour fermer le circuit de traitement une câble (8) avec des oeillets (9, 9') de contact à ressort est arrangée entre les deux contacts à bouton-pression (7, 7') des emplâtres à électrode d'électrocardiographie.

2. Dispositif d'emplâtre selon la revendication 1, caractérisé en ce qu'un isolement (3, 3') est prévu au bord des métaux (2, 1 et 1', 2') en forme d'un disque.

3. Dispositif d'emplâtre selon la revendication 1 ou 2, caractérisé en ce qu'une résistance électrique variable est insérée dans la câble (8).

4. Dispositif d'emplâtre selon une des revendications précédentes caractérisé en ce que la câble (8) est élastiquement extensible.

5. Dispositif d'emplâtre selon une des revendications précédentes, caractérisé en ce que les disques métalliques (1, 2') de la premiére électrode en forme d' emplâtre et de la deuxième électrode d'emplâtre tournés vers la peau sont composés en le même matériau de haute qualité qui est compatible avec la peau et conducteur à l'électricité.

6. Dispositif d'emplâtre selon une des revendications précédentes, caractérisé en ce que ce dispositif comprend un porteur imbibé d'un électrolyte, de préférence un papier brouillard mouillé imbibé avec de la soude, qui est arrangé entre les disques métalliques (1, 2') tournés vers la peau et la peau.

7. Dispositif d'emplâtre selon une des revendiations précédentes, caractérisé en ce que le dispositif d'emplâtre est composé de plus de deux électrodes en forme d'emplâtre.
